Europäisches Patentamt

European Patent Office

Office européen des brevets

(11) Publication number: **0 364 281**

**A2**

(12) # EUROPEAN PATENT APPLICATION

(21) Application number: **89310499.2**

(51) Int. Cl.5: **A61K 6/06**

(22) Date of filing: **12.10.89**

(30) Priority: **14.10.88 US 257543**

(43) Date of publication of application:
**18.04.90 Bulletin 90/16**

(84) Designated Contracting States:
**DE FR GB IT**

(71) Applicant: **WARDEN-PITTS DENTAL LABS, INC.**
**104 Kenner Avenue Suite 302**
**Nashville Tennessee 37205(US)**

(72) Inventor: **Philip Gordon, Pitts**
**401 Brooksboro Terrace**
**Nashville Tennessee 37217(US)**
Inventor: **Charles Leon, Warden**
**6121 Nolensville Road**
**Nashville Tennessee 37205(US)**

(74) Representative: **Hulse, Thomas Arnold et al**
**Hulse & Co. Cavendish Buildings West Street**
**Sheffield S1 1ZZ(GB)**

(54) Spray opaque composition for coating dental appliances.

(57) A spray opaque composition for coating restorations comprises 10-30 weight % opaque material, 10-30 weight % restoration wetting agent, 10-30 % suspending agent for maintaining opaque material in suspension for spraying and 10-70% propellant. The wetting agent is an alcohol selected from a group consisting of methyl, ethyl, isopropyl and any mixtures thereof. Preferably, the wetting agent is anhydrous. The suspending agent is trichlorotrifluoroethane. The propellant is selected from a group consisting of isobutane, butane, chlorodifluoromethane and any mixtures thereof.

EP 0 364 281 A2

## SPRAY OPAQUE COMPOSITION FOR COATING DENTAL APPLIANCES

Technical Field

The present invention relates generally to the dental field and, more particularly, to an aerosol spray opaque formulation that is easy to use while providing even and consistent application of porcelain to metal restorations.

Background of the Invention

For years, dentists have practiced the art of replacing missing teeth of their patients with metal restorations. In order to improve the life of the restorations as well as maximize aesthetic appeal, the metal restorations are coated with opaque or porcelain to provide a color substantially matching the color of the other teeth of the patient.

One technique uitlized to apply opaque or porcelain to a metal restoration is by brush or instrument. An opaque is chosen as closely as possible matching the color and shade of the patient's teeth. The opaque is then mixed with an opaque liquid to a creamy consistency. The opaque liquid allows complete wetting of the surface of the restoration so that the opaque will properly attach and coat. The restoration is carefully cleaned and all dirt and oil is removed. The restoration is then dampened with distilled water or opaque liquid to aid in wetting. Next, the brush is dipped into the opaque mixture and a thin coating of opaque is applied to the restoration by gently tapping the brush and restoration. Once completely covered, the restoration is fired to dry and set the opaque. An opaque mixture of thicker consistency is then made and the application and firing procedure repeated to add a second, thicker coating.

While it is possible to apply a relatively smooth or consistent coating of opaque with the brush or instrument technique, it does require a great deal of experience and skill. For example, if the opaque mixture is too thin, the opaque is hard to control. This leads to uneven, inconsistent coating that may also exhibit poor bonding characteristics. In contrast, if the opaque mixture used is too thick, the coating may be too thick. This can lead to bubbling during firing and poor bonding from incomplete wetting of the allow surface.

Insufficient wetting may also result if the technician forgets to dampen the restoration before applying the opaque. This can lead to spots without bond or blistering of the opaque. Further, those not experienced may try to blot on the opaque with the brush rather than using a tapping action. Disadvantageously, blotting has a tendency to negate the wetting action and actually serves to lift opaque from the restoration.

The proper control of the firing cycle is also vital. Too rapid heating of the opaque causes sudden evaporation of opaque liquid resulting in poor bonding through bubbling or lift off of the opaque. Careful control of the temperature is also critical in the final stages of firing in order to obtain the desired "eggshell" glisten that as closely as possible matches the patient's actual tooth enamel. Overfiring produces a glassy appearance that adversely affects the coloring of the restoration. Conversely, underfiring produces a loss of vitality in the final shade.

Thus, as should be appreciated, the brush or instrument approach is very technique sensitive, requiring a highly skilled and experienced artisan for best results. Not only must a highly paid technician be engaged to complete this work but often the coating processes must be repeated or completely reinitiated because of the many variables that can prevent satisfactory completion of the coating process. These factors significantly add to the cost of a coated restoration.

it is clear from the above that a new and easier method or coating restorations and/or crowns with the opaque is needed. In response to this need, the air brush technique was developed. In this technique, the proper opaque is selected and mixed with opaque liquid in an air brush bottle. The air brush is attached and test spraying is conducted. The consistency of the mixture is then adjusted as necessary until the desired spraying action is acquired. A cleaned restoration is then gently warmed and the opaque is applied in a light spray coating. Firing, as described above, is then utilized to dry and set the opaque. A thicker consistency of opaque is then mixed for spraying the second coating. After application of the second coating, the opaque is then again fired.

While the air brush procedure is superior to the brush or instrument technique in providing a smooth, consistent coating, it is not without its disadvantages. Considerable equipment at significant expense is required. There are also no standards. Essentially, the air brush setups are each customed built by technicians to spray opaque. Thus, the opaque mixtures are not standardized but must be custom made specifically for each particular air brush setup. Of course, since spray coatings vary from one air brush to another, firing times and temperatures also must be individually tailored and carefully controlled for best results. A need, therefore, is identified for an improved opaquing technique that both simplifies the application of opaque in an even and consistent coating while also standardizing the procedure and reducing the cost of producing coated restorations.

Summary of the Invention

It is accordingly a primary object of the present invention to provide a spray opaque composition for overcoming the above-described limitations and disadvantages of the prior art and filling the need for an improved method of opaquing.

Still another object of the present invention is to provide a spray opaque composition that is easy to use, allowing the application of a uniform layer of opaque to a restoration or crown even by an individual with a minimal amount of experience or skill with the procedure.

Yet another object of the present invention is to provide a spray opaque composition in aerosol form that produces a consistent spray pattern without spattering or dripping to allow the consistent application of a uniform layer of opaque to a restoration. Advantageously, the application of a uniform layer allows greater standardization of firing times and temperatures so as to further assure proper results.

An additional object of the present invention is to provide a spray opaque composition that is premixed to the desired consistency for immediate, convenient use while also providing the best possible results in applying opaque to dental restorations.

Still another object of the invention is to provide a spray opaque in aerosol form that is relatively inexpensive, that produces consistent coating results without being particularly technique sensitive and that is disposable so as to reduce both lab time and clean up costs.

Additional objects, advantages and other novel features of the invention will be set forth in part in the description that follows and in part will become apparent to those skilled in the art upon examination of the following or may be learned with the practice of the invention.

In satisfaction of the foregoing objects and advantages, there is provided by this invention a spray opaque composition for coating restorations, crowns or other dental applicances. The composition comprises by weight 10-30% opaque material, 10-30% wetting agent, 10-30% suspending agent and 10-70% propellant.

More preferably, the composition comprises 12.5-25% opaque material, 12.5-25% wetting agent, 12.5-25% suspending agent and 25-62.5% propellant.

The opaque material may be a natural or man made feldspar or a feldspar mixture. Opaque material is available from a number of manufacturers. This is the same opaque material currently utilized by technicians practicing the brush or instrument and air brush application techniques.

The wetting agents best suited for utilization in the composition are those that do not react with other components of the composition and that are fairly easy to volatilize yet do not represent a significant safety hazard during drying of the restoration after opaque application. Preferably, an anhydrous alcohol is uitlized. The preferred alcohols include methyl, ethyl and isopropyl or any mixtures thereof.

The suspending agent of the present invention is effective in maintaining the opaque material and suspension so as to provide smooth, even and consistent spraying action without clogging, sticking or spattering. A compound particularly effective as an opaque suspending agent is trichlorotrifluoroethane.

The propellant utilized is selected from a group consisting of isobutane, butane, chlorodifluoromethane and any mixtures thereof. While spray pressures may range between 20-100 psig, for best results and the most consistent spray characteristics, the original composition is packaged at a pressure in the range of 80-100 psig. This may be achieved by utilizing a mixture of chlorodifluoromethane with isobutane and/or butane.

Detailed Description of the Invention

To achieve the foregoing and other objects and in accordance with the purposes of the present invention as described herein, a spray opaque composition is provided. The composition comprises in weight 10-30% opaque material, 10-30% wetting agent, 10-30% suspending agent and 10-30% propellant.

In a more preferred embodiment, the spray opaque composition comprises by weight 12.5-25% opaque material, 12.5-25% wetting agent, 12.5-25% suspending agent and 25-62.5% propellant.

The opaque material may be in the form of a natural or man made feldspar or a feldspar mixture. Such material is available from a number of different manufacturers under varying designations. Some sources of opaque material include Ceramco, Inc. of East Windsor, New Jersey, Williams Dental Company of Buffalo, New York, Dentsply, Inc. of York, Pennsylvania and Vident, Inc. of Baldwin Park, California.

Because of varying percentages of, for example, man made and natural feldspar in the various opaque material formulations available from these manufacturers, the intrinsic characteristics of the opaque material also vary. As a result, the particular weight percentages of the individual components of the spray opaque composition may, of course, need to be adjusted across the range as discussed above to provide proper and consistent results.

The wetting agent utilized in the spray opaque composition of the present invention is preferably an alcohol. Anhydrous isopropyl alcohol is a particularly useful wetting agent. Not only does it provide very good wetting action, but it is water-free. Therefore, there is no freezing of water droplets as the composition cools during spraying. This is a significant feature since freezing water droplets cause the formation of opaque platelets that lead directly to spotting and an inconsistent coating. It should be recognized, however, that alcohols other than anhydrous isopropyl alcohol could be utilized. For example, both methyl alcohol and ethyl alcohol are also effective wetting agents. So are any mixtures of methyl, ethyl and isopropyl alcohol. In any event, use of any of these alcohols in anhydrous form is preferred.

The use of an appropriate suspending agent is also important to providing the desired characteristics for the spray opaque composition of the present invention. A number of problems have prevented past attempts to produce an effective aerosol opaque spray from succeeding. These include insufficient wetting of the restoration, as well as spray orifice clogging, valve sticking and opaque spattering. All these problems lead to completely unacceptable coatings. Advantageously, the present spray opaque composition avoids each of these difficulties.

More specifically, the suspending agent trichlorotrifluoroethane provides additional wetting action to the composition. Advantageously, it has also been found particularly effective in lifting any traces of oil from the surface of the metal restoration. This is an important feature since minute traces of oil may be left by the fingers when handling a restoration after the restoration has undergone a thorough cleaning process. Since any traces of oil left by handling are lifted from the surface of the restoration by the action of the trichlorotrifluoroethane, the possibility of this oil causing improper attachment and lifting of opaque from the restoration is avoided.

Trichlorotrifluoroethane is also very effective in maintaining the opaque material in suspension with a minimum amount of shaking to remix the opaque composition in the spray container. Advantageously, with the opaque material maintained in suspension, smooth, even and consistent spraying action is provided without problems of clogging, sticking or spattering. The trichlorotrifluoroethane is available from Dupont de Nemours under the trademark Freon TF.

The propellant utilized is also very important in providing the desired spraying action. More specifically, the spray composition must be pressurized sufficiently to maintain the flow of the opaque material through the valve mechanism and spray orifice without clogging. The pressure, however, must not be so great as to make control of the spray pattern difficult.

Propellants that may be utilized in the spray opaque composition of the present invention are selected from a group consisting of isobutane, butane, chlorodifluoromethane and any mixtures thereof. While a pressure range between 20 and 100 psig may be utilized, best results and consistent spray characteristics are obtained where the original composition is packaged at a pressure in the range of 80-100 psig. This is achieved by utilizing a mixture of chlorodifluoromethane with isobutane and/or butane. The butane and isobutane hydrocarbon propellants are available, for example, from the Phillips Chemical Company under the designations A-17 and A-31, respectively. The chlorodifluoromethane propellant is available from Dupont de Nemours under the trademark Freon 22.

In accordance with the requirements for the spray composition set forth above, a particularly effective composition includes 24% opaque material, 20.5% anhydrous methyl, ethyl and/or isopropyl alcohol, 24.5% trichlorotrifluoroethane suspending agent and 35% propellant. For best spray characteristics, the 35% propellant is an even mixture of butane and chlorodifluoromethane (i.e. 17.5% butane and 17.5% chlorodifluoromethane). At these percentages there is sufficient alcohol in the composition to provide good wetting of the restoration being sprayed. Thus, there is good adherence between the opaque material and the surface metal of the restoration. There is also sufficient suspending agent to both aid in the wetting and maintain the opaque material in suspension without excessive shaking of the aerosol can. Further, there is sufficient spray pressure to prevent clogging and valve sticking. The pressure, however, is not excessive so that a good spray pattern is produced that may be both easily and accurately controlled. Thus, the composition advantageously exhibits the desired smooth, even and consistent spray characteristics that allow even relatively unskilled and inexperienced technicians to apply a uniform opaque or porcelain coating to dental applicances.

The following examples are presented to further illustrate the invention but it should be recognized that the invention is not to be considered limited thereto.

Example 1


Spray Opaque Composition


22.8 grams of Ceramco A-2 shade opaque material was added to 19.475 grams of anhydrous isopropyl alcohol and 19.475 grams of trichlorotrifluoroethane in a 202 x 214 lined aerosol spray container manufactured by Continental Can Company. These materials were thoroughly mixed and the spray container crimped and sealed. The container was then charged with propellant. More specifically, 16.625 grams of butane and 16.625 grams of chlorodifluoromethane was added to the contents of the aerosol spray container. This provided the aerosolized spray opaque composition with a pressure of approximately 96 psig at 70° F.

The aerosol spray container was equipped with a customized spray valve assembly available from Precision Valve Company of Yonkers, New York. The specifics of the particular spray valve assembly utilized in this example and those to follow are:

Actuator button: .025" aqua NMBU Kosmos (PVC part no. 21-9126).

Stem: .024" ringed, barbed, 630" OAL (PVC part no. 04-0562-42)

Stem gasket: Neoprene (PVC part no. 05-0330)

Spring: .020" SS (PVC part no. 06-6044)

Body: .062 X .013" VT .412" Deep (PVC part no. 07-3428)

Mounting cup: conical, open top/bottom, dimpled, bonded PE (PVC part no. 32-8300-71)

Dip tube: .050" CAP (PVC part no. 09-3520)


Application Procedure


An aerosolized spray opaque composition described above was next applied to a series of dental restorations. Each of the restorations was carefully cleaned of all dirt and oil. The spray container was then shaken vigorously for approximately 3 minutes to bring the opaque into suspension and fully mix the contents. Next, the spray container and restoration were both brought into position for application of the spray opaque to the metal surface of the restoration. During application, it was noted that droplets of opaque appeared on the restoration when the restoration and spray tip were held too close together during spraying. A similar result occurred when the spray container was nearly empty. Some puddling of opaque on a restoration also occurred when a sufficient distance was not maintained between the spray tip and the restoration during application. For best results, it was found that a distance of approximately 6 to 8 inches needs to be maintained between the spray tip and the restoration during spraying.

Once a slight sheen of opaque material appeared over the entire surface of each restoration, the restoration were fired in accordance with the instructions of the opaque manufacturer. After initial firing and setting of the first coat, a second coat was applied and fired in the same manner. An opaque coating of execellent quality resulted.


Example 2


In this example 22.8 grams of Vita A-2 shade opaque material is substituted for the 22.8 grams of Ceramco A-2 shade opaque material utilized in Example 1. All other components and processing are the same as presented in Example 1.


Example 3


25.0 grams of Williams Dental Company Wob-1 opaque material was added to 20.0 grams of anhydrous isopropyl alcohol and 20.0 grams of trichlorotrifluoroethane in a 200 x 214 lined aerosol spray container manufactured by Continental Can Company. These materials were thoroughly mixed and the spray container crimped and sealed. The container was then charged with propellant. More specifically, 17.5 grams of butane and 17.5 grams of chlorodifluoromethane were added to the contents of the aerosol spray container.


Example 4


20.58 grams of Williams Dental Company WOB-1 opaque material was added to 20.58 grams of anhydrous isopropyl alcohol and 20.58 grams of trichlorotrifluoroethane in a 202 x 214 lined aerosol spray container manufactured by Continental Can Company. These materials were thoroughly mixed and the spray container crimped and sealed. The container was then charged with propellant. More specifically, 16.625 grams of butane and 16.625 grams of chlorodifluoromethane were added to the contents of the aerosol spray container.

In summary, numerous benefits result from employing the concepts of the present invention. An aerosolized spray opaque is provided that is very convenient to use. The spray opaque composition produces a consistent spray pattern without spattering or dripping. It allows a consistent application of a uniform layer of opaque to a restoration even by an individual with a minimum amount of experience or skill with the procedure.

**Claims**

1. A spray opaque composition for coating restorations and dental applicances, being characterized by weight percent:
10.0% to 30.0% opaque material;
10.0% to 30.0% wetting agent;
10.0% to 30.0% suspending agent for maintaining opaque material in suspension for spraying; and
10.0% to 70.0% propellant.

2. The spray opaque composition set forth in Claim 1, wherein said wetting agent is an alcohol selected from a group consisting of methyl alcohol, ethyl alcohol, isopropyl alcohol and any mixtures thereof.

3. The spray opaque composition set forth in Claim 1, wherein said suspending agent is trichlorotrifluoroethane.

4. The spray opaque composition set forth in Claim 1, wherein said propellant is a hydrocarbon propellant.

5. The spray opaque composition set forth in Claim 1, wherein said propellant is selected from a group consisting of isobutane, butane, chlorodifluoromethane and any mixtures thereof.

6. The spray opaque composition set forth in Claim 1, wherein said composition in weight percent includes 15% to 30% opaque material, 15% to 30% alcohol wetting agent, 15% to 30% suspending agent and 10% to 55% propellant.

7. The spray opaque composition set forth in Claim 1, wherein said composition in weight percent includes 20%-28% opaque material, 20-28% alcohol wetting agent, 20-28% suspending agent and 16-40% propellant.

8. The spray opaque composition set forth in Claim 1, wherein said composition in weight percent includes substantially 24.0% opaque material, 20.5% alcohol wetting agent, 20.5% suspending agent and 35.0% propellant.

9. The spray opaque composition set forth in Claim 8, wherein said alcohol wetting agent is isopropyl alcohol, said suspending agent is trichlorotrifluoroethane and said propellant is 17.5% butane and 17.5% chlorodifluoromethane.

10. A spray opaque composition for coating restorations and dental applicances, being characterized by weight percent:
12.5% to 25.0% opaque material;
12.5% to 25.0% alcohol wetting agent selected from a group consisting of methyl alcohol, ethyl alcohol, isopropyl alcohol and any mixtures thereof;
12.5% to 25.0% suspending agent for maintaining opaque material in suspension during spraying; and
25.0% to 62.5% propellant selected from a group consisting of butane, isobutane,, chlorodifluoromethane and any mixtures thereof.

11. The spray opaque composition set forth in Claim 10, wherein said suspending agent is trichlorotrifluoroethane.